# EUROPÄISCHE PATENTSCHRIFT

(11) **EP 0 620 013 B1**
(45) Veröffentlichungstag und Bekanntmachung des Hinweises auf die Patenterteilung: **29.06.2005**
(21) Anmeldenummer: 94104064.4
(22) Anmeldetag: 16.03.1994
(51) Int. Cl.: A61L 2/18, A01N 33/04

(54) **Desinfektionsmittel für Sammeltoilettensysteme und ähnliche Anlagen**
Desinfectant for toilet systems with holding tanks and similar equipment
Désinfectant pour systèmes de toilettes à réservoir et autres équipements

(30) Priorität: 15.04.1993 DE 4311840
(43) Veröffentlichungstag der Anmeldung: 19.10.1994
(73) Patentinhaber: Bode Chemie GmbH & Co., 22525 Hamburg (DE)
(72) Erfinder: Anker, Willem, Dr., D-25474 Ellerbek (DE); Lux, Dorit, D-22393 Hamburg (DE); Ostermeyer, Christiane, D-22559 Hamburg (DE); Winter-Extra, Susanne, Dr., D-22767 Hamburg (DE)
(74) Vertreter: Schaeffer, Michael

(56) Entgegenhaltungen:
- EP-A- 0 333 143
- EP-A- 0 343 605
- US-A- 4 229 408

## Beschreibung

Die Erfindung betrifft Desinfektionsmittel für Sammeltoilettensysteme und vergleichbare Anlagen.

Der immer schneller werdende Luftverkehr läßt geographische Entfernungen zusammenschrumpfen und rückt die Seuchengebiete der Tropen und Subtropen sozusagen sehr viel näher an die nördliche Hemisphäre heran. Während früher die See- und Uberlandfahrten mit Schiff oder Eisenbahn aus diesen Gebieten in die nördlichen Länder fast immer länger dauerten als die Inkubationszeit von hochgradig infektiösen Erkrankungen wie Cholera, Pocken oder Gelbfieber, ist dies durch den sich fortwährend intensivierenden Luftverkehr längst nicht mehr der Fall.

Eine potentielle Gefahrenquelle stellen in dieser Situation die Rezirkulations- und Vakuumtoiletten der Passagierflugzeuge dar. Bei diesen Umwälztoiletten wird nämlich die ursprüngliche Spülflüssigkeit mit den Ausscheidungen vermengt, durch Filter aus dem Fäkaltank hochgepumpt und immer wieder zum Spülen der Toilettenbecken benutzt. Dabei kann nicht ausgeschlossen werden, daß durch Verspritzen oder Aerosolbildung die Toilettenoberfläche, der Raum und ggf. Körper oder Kleidung eines Toilettenbenutzers kontaminiert werden. Diese Situation ist vom seuchenhygienischen Standpunkt aus äußerst bedenklich, da eine Vielzahl von Krankheiten wie Typhus, Paratyphus, Ruhr, Cholera oder Salmonellose durch menschliche Ausscheidungen übertragbar sind. Ein Übergreifen von Infektionen auf andere Fluggäste ist daher bei Anwesenheit eines Erkrankten oder Ausscheiders kaum zu vermeiden. Besonders risikoreich ist dies bei der Cholera, die häufig eine Inkubationszeit von nur wenigen Stunden aufweist, so daß Personen, die sich kurz vor oder nach dem Start infiziert haben, noch während eines Langstreckenfluges an Brechdurchfällen erkranken und so zur weiteren Ausbreitung beitragen können.

In Flugzeugtoiletten sollten daher zwingend nur Stuhldesinfizientien zum Zuge kommen, die sowohl innerhalb ausreichend kurzer Zeit zum Schutz der nachfolgenden Benutzer als auch bei voller Tankaus- und Stuhlbelastung zum Schutze des Entsorgungspersonals wirken. Entsprechende Vorschriften lassen sich beispielsweise aus der Aerospace Material Specification AMS 1476 A, entnehmen.

Zur Zeit werden für geschlossene Toilettensysteme Desinfektionsmittel auf Aldehydbasis, ggf. mit Zusatz von Quats oder ausschließlich quartäre Ammoniumverbindungen eingesetzt (vergl. z.B. US-A-4 229 408). Die Verwendung von Aldehyden oder Aldehydabspaltern weist eine Reihe von Nachteilen auf. Diese Verbindungen sind nicht besonders gut hautverträglich und können zu Sensibilisierungen führen. Außerdem denaturieren und koagulieren sie Proteine und werden dadurch schnell in der Wirk-samkeit beeinträchtigt. Schließlich kommt noch hinzu, daß Aldehyde meistens stark riechen und daher zu unerwünschten Geruchsbelästigungen führen. Sowohl für Aldehyde wie auch für Quats trifft zu, daß die Materialverträglichkeit außerdem zu wünschen übrigen läßt und daß auch die biologische Abbaubarkeit häufig nicht optimal ist und zu Beanstandungen durch Behörden Veranlassung geben kann.

Ähnliche Probleme stellen sich überall dort, wo menschliche oder tierische möglicherweise kontaminierte Ausscheidungen über eine längere Zeitspanne in Sammelbehältern untergebracht werden müssen, weil ihre Entsorgung nicht sofort möglich ist. Hierzu gehören die sogenannten mobilen Toiletten, wie sie beispielsweise bei Camping und Kleingärten, aber auch als Toiletten für Bauhandwerk oder bei Sportveranstaltungen oder Volksfesten benutzt werden. Desgleichen gehören in diesen Problemkreis Sammelbehälter für Monatshygienebedarf, wie sie in vielen öffentlich zugänglichen Toiletten Verwendung finden. Zwar ist in diesen Fällen die Anzahl derjenigen Personen, die möglicherweise einer Infektion ausgesetzt sind, in der Regel geringer als bei vollbesetzten Passagierflugzeugen; trotzdem darf auch in diesem Zusammenhang nicht die Gefahr einer Infektion als solcher und/oder die Möglichkeit der Weiterverbreitung von Infektionen übersehen werden.

Eine ähnliche Problematik besteht hinsichtlich tierischer Ausscheidungen, wie sie beispielsweise in großen Mengen bei der Schweine- oder Hühnerzucht anfallen. Soweit Ausscheidungen gesammelt und gelagert werden oder an Ort und Stelle verbleiben, bevor sie entfernt werden, besteht die Gefahr, daß einerseits nicht nur einzelne infizierte Tiere eine Infektion auf den ganzen Bestand übertragen können, sondern auch die Gefahr, daß sich das in solchen Betrieben beschäftigte Personal infizieren kann, wenn die vorhandenen Keime sowohl menschen- als auch tierpathogen sind. Obgleich dieser Problemkreis erst seit kurzem größere Aufmerksamkeit gefunden hat, stimmen die Seuchenhygieniker in der Beurteilung der Relevanz überein.

Es besteht daher noch ein Bedarf nach Desinfektionsmitteln für Sammeltoilettensysteme und ähnliche Anlagen, die die oben geschilderten Nachteile nicht aufweisen.

Erfindungsgemäß werden nunmehr wässrige Desinfektionsmittellösungen mit einem pH-Wert von 5-10 vorgeschlagen, die dadurch gekennzeichnet sind, daß sie Salze von Aminen der folgenden Formel I und/oder II enthalten, 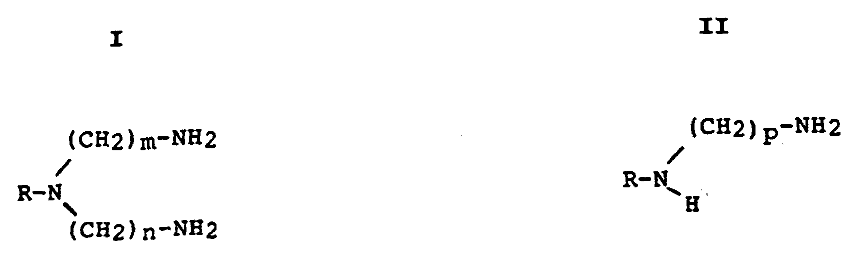 in denen R eine gerade oder verzweigte Alkyl- oder Alkylenkette mit 6-22 C-Atomen und n und m zusammen einen Wert von 4 bis 12 und p einen Wert von 2 bis 12 bedeuten.

Diese Amine mit relativ langen Alkylketten sind an sich bekannt. Verbindungen mit mehr als 4 C-Atomen in der Alkyl- oder Alkylenkette werden als Fettamine bezeichnet und weisen eine inhärente antimikrobielle Wirksamkeit auf (vergl. z.B. EP-A-0 343 605). Auch bei Verbindungen mit kürzeren Ketten ist eine keimabtötende Wirksamkeit feststellbar, die aus ihrer hohen Eigenalkalität resultiert und aus der Fähigkeit aller dieser Amine, Oberflächenspannungen zu reduzieren.

Die Herstellung dieser Amine kann beispielsweise nach dem in der DE-C-21 52 787 beschriebenen Verfahren erfolgen.

Die erfindungsgemäß eingesetzten Amine zeigen eine überraschend gute antimikrobielle Wirksamkeit bei gleichzeitig sehr guter Wasserlöslichkeit, wobei die germizide Wirkung weder durch übliche technologisch bedingte Zusätze noch unter Eiweißbelastung nennenswert nachläßt. Völlig überraschend war aber die Tatsache, daß bei Absinken des pH-Wertes durch Salzbildung oder Einstellung mit einer physiologisch unbedenklichen Säure die Wirksamkeit sogar noch gesteigert wird, während gleichzeitig die Materialverträglichkeit auch gegenüber Metallen wie Aluminium erhöht wird, die sonst durch die Alkalität der Verbindungen als nicht optimal einzustufen war.

Vorzugsweise werden Amine eingesetzt, bei denen in der allgemeinen Formel R 8-18 C-Atome enthält und n + m einen Wert von 6 und p einen Wert von 3 aufweisen. Die Amine liegen als Salze entweder anorganischer Säuren vor, wobei die physiologisch unbedenklichen Chloride, Sulfate oder Phosphate bevorzugt werden oder sie können auch mit organischen Säuren umgesetzt sein, und zwar vorzugsweise mit Ameisensäure, Essigsäure, Propionsäure, Oxalsäure, Zitronensäure, Apfelsäure, Milchsäure oder ähnlichen unbedenklichen Säuren. Der pH-Wert in wäßriger Lösung liegt zwischen 10-5 und vorzugsweise zwischen 9-7, da in diesem Bereich eine hervorragende Materialverträglichkeit gegeben ist. Die Verbindungen können in wäßriger Lösung eingesetzt werden, es können aber auch wäßrig-alkoholische Lösungen zum Einsatz kommen, wobei als Alkohole meist Ethanol, Isopropanol, Glykole oder Glycerin zugesetzt werden. Anstelle von Alkoholen können gegebenenfalls auch physiologisch unbedenkliche Ester oder Ether als Lösungsmittel eingesetzt werden.

Die Konzentration in wäßriger oder teilweise wäßriger Lösung an Aminen kann zwischen etwa 0,01 %, der Wirksamkeitsuntergrenze, bis zur Löslichkeitsgrenze der jeweiligen Verbindung gehen, aber meist werden 1-30 und vorzugsweise 5-15 Gew.-% eingesetzt. Auch pastenförmige Zubereitungen lassen sich in an sich bekannter Weise herstellen durch Zugabe von Verdickungsmitteln zu wäßrigen oder teilweise wäßrigen Lösungen.

Den Zubereitungen können die üblichen Zusatzstoffe zugesetzt werden wie beispielsweise Tenside, Korrosionsinhibitoren, Entschäumer, Netzmittel, Komplexierungsmittel, Verdickungsmittel, Farbstoffe, Parfüms und ähnliches.

Die Anwendung der erfindungsgemäßen Desinfektionsmittel erfolgt in einer Konzentration von mindestens 0,005-12 Gew.-%, bezogen auf den Gesamtamingehalt.

Die erfindungsgemäßen Mischungen zeigen eine hohe bakterizide, viruzide und fungizide Wirksamkeit und zeichnen sich gleichzeitig durch Hautverträglichkeit, sehr geringe Toxizität, geringe Korrosivität und gute Materialverträglichkeit, biologische Abbaubarkeit, Lagerstabilität und geringe Geruchsbelästigung aus. Bei einen pH-Wert zwischen 7 und 9 sind auch 15%-ige Lösungen bei Raumtemperatur und bei Temperaturen bis 50°C nicht korrosiv gegenüber Stahl, und Metallen, Aluminium und lackiertem Aluminium, elastischen Dichtungsmassen, Gumme, Kautschuk, PMMA und anderen Kunststoffen.

Die Erfindung wird im folgenden anhand der Beispiele näher erläutert:

### Beispiel 1

| | |
|---|---|
| N,N-Bis-(3-aminopropyl)-laurylamin | 7.5% |
| Natriumethylendiamintetraacetat | 2.8% |
| Fettalkoholethyxylat (CB-C12 mit 8-12 EO) | 5.0% |
| Citronensäure | 2.0% |
| Wasser | 92.4% |
| Parfüm | 0.3% |

### Beispiel 2

| | |
|---|---|
| N,N-Bis-(3-aminopropyl)-laurylamin | 12.0% |
| Fettalkoholethoxylat (C16-C-18 mit 25 EO) | 3.0% |
| Ameisensäure, konzentriert | 4.8% |
| Butindiol | 1.8% |
| Entschäumer | 0.1% |
| Farbstoff E 131 | 0.5% |
| Wasser | 77.7% |

### Beispiel 3

| | |
|---|---|
| N,N-Bis-(3-aminopropyl)-laurylamin | 2.8% |
| Lauryl-propyl-diamin | 2.2% |
| Natriumlaurylsulfat | 0.56 |
| Salzsäure, 10% | 2.0% |
| 1,2-Propylenglykol | 10.0% |
| Wasser | 80.44% |
| Verdicker | 2.0% |

### Beispiel 4

| | |
|---|---|
| N,N-Bis-(3-aminopropyl)-laurylamin | 58.0% |
| Essigsäure, konzentriert | 27,4% |
| Polyglyko120,000 | 14,6% |

## Patentansprüche

1. Wäßrige oder teilweise wäßrige Desinfektionsmittelösung mit einem pH-Wert von 5-10 für Sammeltoiletten und ähnliche Einrichtung, wobei die Lösung Salze von Aminen der allgemeinen Formeln I oder II enthält, 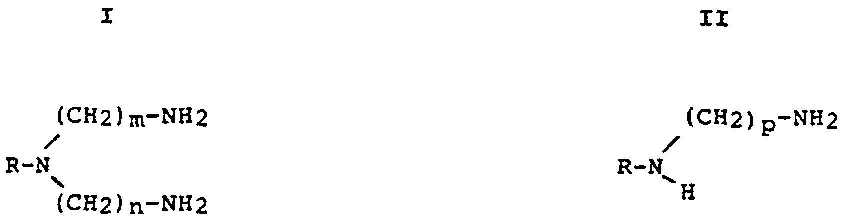 in denen R einen gradkettigen oder verzweigten Alkyl- oder Alkylenrest mit 6 - 22 C-Atomen aufweisen, wobei n und m zusammen einen Wert von 4 bis 12 und p einen Wert von 2-12 bedeuten.

2. Desinfektionsmittellösung nach Anspruch 1, **dadurch gekennzeichnet, daß** sie Amine der allgemeinen Formel I zusammen mit Aminen der allgemeinen Formel II enthalten.

3. Desinfektionsmittellösung nach Anspruch 1 oder 2, **dadurch gekennzeichnet, daß** sie Amine enthalten, bei denen in den allgemeinen Formeln R 8 bis 18 C-Atome und n + m zusammen einen Wert von 6 und p einen Wert von 3 bedeuten.

4. Desinfektionsmittellösung nach Anspruch 1 bis 3, **dadurch gekennzeichnet, daß** sie i Form von Salzen physiologisch verträglicher anorganischer Säuren vorliegen.

5. Desinfektionsmittellösung nach Anspruch 1 bis 4, **dadurch gekennzeichnet, daß** die Amine als Salze organischer Säuren vorliegen.

6. Desinfektionsmittelösung nach Anspruch 1 bis 5, **dadurch gekennzeichnet, daß** die wäßrige Lösung einen pH-Wert von 9-7 aufweist.

7. Desinfektionsmittellösung nach Anspruch 1 bis 6, **dadurch gekennzeichnet, daß** sie in Wasser als Lösungsmittel oder in im wesentlichen wäßrig-alkoholischen Lösungen vorliegen.

8. Desinfektionsmittellösung nach Anspruch 7, **dadurch gekennzeichnet, daß** die wäßrig-alkoholischen Lösungsmittel Ethanol, Isopropanol, n-Propanol, Glykole oder Glycerin enthalten.

9. Desinfektionsmittellösung nach Anspruch 1 bis 8, **dadurch gekennzeichnet, daß** die Konzentration der Aminsalze etwa 0,01 Gew.-% bis Löslichkeitsgrenze beträgt.

10. Desinfektionsmittellösung nach Anspruch 1 bis 9, **dadurch gekennzeichnet, daß** sie zusätzlich Tenside und/oder Korrosionsinhibitoren und/oder Entschäumer und/oder Netzmittel und/oder Komplexierungsmittel und/oder Verdicker und/oder Farbstoffe und/oder Parfümstoffe enthalten.

11. Verwendung von Desinfektionsmittellösungen nach Anspruch 1 bis 10 für Sammeltoilettensysteme, **dadurch gekennzeichnet, daß** die Anwendungskonzentration mindestens 0,005 bis etwa 5 Gew.-%, bezogen auf den Gesamtamingehalt, beträgt.

12. Verwendung von Desinfektionsmittellösungen nach Anspruch 1 bis 10 für Sammelbehälter für tierische Ausscheidungen, **dadurch gekennzeichnet, daß** die Anwendungskonzentration mindestens 0,005 bis etwa 12 Gew.-%, bezogen auf den Gesamtamingehalt, beträgt.

13. Desinfektionsmittelösung nach Anspruch 4, **dadurch gekennzeichnet, daß** sie Chlorid-, Sulfat- oder Phosphat-Anione aufweist.

14. Desinfektionsmittellösung nach Anspruch 5, **dadurch gekennzeichnet, daß** die Amine als Salze der Ameisensäure, Essigsäure, Propionsäure, Oxalsäure, Zitronensäure, Apfelsäure oder Milchsäure vorliegen.

15. Desinfektionsmittellösung nach Anspruch 9, **dadurch gekennzeichnet, daß** die Konzentration der Aminsalze 5 - 15 Gew.-% beträgt.

## Claims

1. An aqueous or partly aqueous disinfectant solution with a pH of 5-10 for waste-collecting toilets and similar installations, wherein the solution contains salts of amines of the general formulae I or II 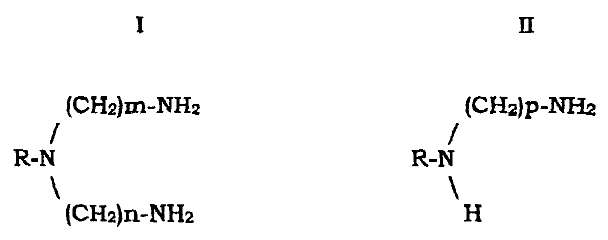 in which R is a straight-chain or branched alkyl or alkylene residue with 6-22 C atoms and wherein n and m together have a value of 4 to 12 and p has a. value of 2 to 12.

2. A disinfectant solution according to claim 1, **characterised in that** it contains amines of the general formula I together with amines of the general formula II.

3. A disinfectant solution according to claim 1 or 2, **characterised in that** it contains amines in which, in the general formulae, R has 8 to 18 C atoms and n + m together have a value of 6 and p has a value of 3.

4. A disinfectant solution according to claims 1 to 3, **characterised in that** it is present in the form of salts of physiologically compatible inorganic acids.

5. A disinfectant solution according to claims 1 to 4, **characterised in that** the amines are present as salts of organic acids.

6. A disinfectant solution according to claims 1 to 5, **characterised in that** the aqueous solution has a pH of 9-7.

7. A disinfectant solution according to claims 1 to 6, **characterised in that** it is present in water as a solvent or in substantially aqueous-alcoholic solutions.

8. A disinfectant solution according to claim 7, **characterised in that** the aqueous-alcoholic solvents contain ethanol, isopropanol, n-propanol, glycols or glycerol.

9. A disinfectant solution according to claims 1 to 8, **characterised in that** the concentration of the amine salts is approximately 0.01 wt.% up to the solubility limit.

10. A disinfectant solution according to claims 1 to 9, **characterised in that** it additionally contains surfactants and/or corrosion inhibitors and/or defoaming agents and/or wetting agents and/or complexing agents and/or thickeners and/or colorants and/or perfumes.

11. Use of disinfectant solutions according to claims 1 to 10 for waste-collecting toilet systems, **characterised in that** the application concentration is at least 0.005 to approximately 5 wt.% based on the total amine content.

12. Use of disinfectant solutions according to claims 1 to 10 for collecting containers for animal excreta, **characterised in that** the application concentration is at least 0.005 to approximately 12 wt.% based on the total amine content.

13. A disinfectant solution according to claim 4, **characterised in that** it contains chloride, sulphate or phosphate anions.

14. A disinfectant solution according to claim 5, **characterised in that** the amines are present as salts of formic acid, acetic acid, propionic acid, oxalic acid, citric acid, malic acid or lactic acid.

15. A disinfectant solution according to claim 9, **characterised in that** the concentration of the amine salts is 5-15 wt.%.

## Revendications

1. Solution d'agent désinfectant, aqueuse ou en partie aqueuse, dont le pH vaut de 5 à 10 et qui est destinée à des toilettes à réservoir et à des dispositifs similaires, laquelle solution contient des sels d'amines de formule générale I ou II : 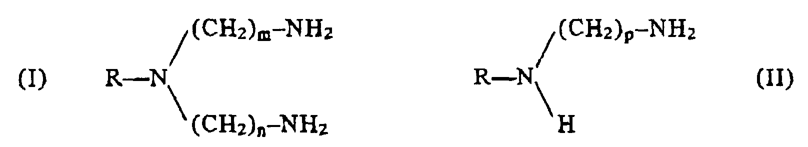 dans lesquelles formules R représente un groupe alkyle ou alcényle à chaîne droite ou ramifiée, comportant de 6 à 22 atomes de carbone, n et m valent ensemble de 4 à 12 et p vaut de 2 à 12.

2. Solution d'agent désinfectant, conforme à la revendication 1, **caractérisée en ce qu'**elle contient des amines de formule générale I, conjointement avec des amines de formule générale II.

3. Solution d'agent désinfectant, conforme à la revendication 1 ou 2, **caractérisée en ce qu'**elle contient des amines chez lesquelles, dans les formules générales, R représente un groupe qui comporte de 8 à 18 atomes de carbone, la somme [n + m] vaut 6 et p vaut 3.

4. Solution d'agent désinfectant, conforme à l'une des revendications 1 à 3, **caractérisée en ce que** les amines s'y trouvent sous la forme de sels d'acides inorganiques physiologiquement compatibles.

5. Solution d'agent désinfectante conforme à l'une des revendications 1 à 4, **caractérisée en ce que** les amines s'y trouvent sous la forme de sels d'acides organiques.

6. Solution d'agent désinfectant, conforme à l'une des revendications 1 à 5, **caractérisée en ce que** le pH de la solution aqueuse vaut de 7 à 9.

7. Solution d'agent désinfectant, conforme à l'une des revendications 1 à 6, **caractérisée en ce que** le solvant est de l'eau ou un solvant essentiellement aqueux-alcoolique.

8. Solution d'agent désinfectant, conforme à la revendication 7, **caractérisée en ce que** le solvant aqueux-alcoolique contient de l'éthanol, de l'isopropanol, du n-propanol, un glycol ou du glycérol.

9. Solution d'agent désinfectant, conforme à l'une des revendications 1 à 8, **caractérisée en ce que** la concentration des sels d'amine vaut d'environ 0,01 % en poids jusqu'à la concentration limite de solubilité.

10. Solution d'agent désinfectant, conforme à l'une des revendications 1 à 9, **caractérisée en ce qu'**elle contient en outre un tensio-actif et/ou un inhibiteur de corrosion et/ou un agent démoussant et/ou un agent mouillant et/ou un agent complexant et/ou un épaississant et/ou un colorant et/ou un agent aromatisant.

11. Emploi d'une solution d'agent désinfectant, conforme à l'une des revendications 1 à 10, dans un système de toilette à réservoir, caracté-risé en ce que la concentration d'application vaut d'au moins 0,005 jusqu'à environ 5 % en poids, pour ce qui est de la teneur totale en amines.

12. Emploi d'une solution d'agent désinfectant, conforme à l'une des revendications 1 à 10, dans une cuve-réservoir à déjections animales, **caractérisé en ce que** la concentration d'application vaut d'au moins 0,005 jusqu'à environ 12 % en poids, pour ce qui est de la teneur totale en amines.

13. Solution d'agent désinfectant, conforme à la revendication 4, **caractérisée en ce qu'**elle contient des anions chlorure, sulfate ou phosphate.

14. Solution d'agent désinfectant, conforme à la revendication 5, **caractérisée en ce que** les amines s'y trouvent sous la forme de sels d'acide formique, d'acide acétique; d'acide propionique, d'acide oxalique, d'acide citrique, d'acide malique ou d'acide lactique.

15. Solution d'agent désinfectant, conforme à la revendication 9, **caractérisée en ce que** la concentration des sels d'amine vaut de 5 à 15 % en poids.
